# EUROPEAN PATENT APPLICATION

(11) **EP 1 980 261 A1**
(43) Date of publication of application: **15.10.2008**
(21) Application number: 08100521.7
(22) Date of filing: 16.01.2008
(51) Int. Cl.: A61K 33/00, A61P 25/00

(54) **Use of helium with oxygen to provide neuroprotection**

(30) Priority: 10.04.2007 EP 07251537
(71) Applicant: Franks, Nicholas Peter, London SW7 2BZ (GB); Maze, Mervyn, London SW10 9NH (GB); Sacristan Martin, Juan Carlos, 28700 Madrid (ES)
(72) Inventor: Franks, Nicholas Peter, London SW7 2BZ (GB); Maze, Mervyn, London SW10 9NH (GB); Sacristan Martin, Juan Carlos, 28700 Madrid (ES)
(74) Representative: Muir, Benjamin M. J.

(57) **Abstract**

Helium is used to provide neuroprotection, in particular in patients having suffered an impact trauma to the head and/or spinal column.

## Description

The present invention relates to the use of helium to provide neuroprotection, in particular neuroprotection against neuronal damage resulting from an impact trauma to the head or spinal column.

The satisfactory treatment of traumatic brain injury (TBI) represents a major unmet clinical need. It has been estimated that each year, in the United States alone, approximately 1.5 million people will sustain TBI. At least 15% will be hospitalized and 3% will die. For approximately 80,000 of those that are hospitalized and survive, the injury will herald the onset of long-term disability. A significant number of those that are injured but are not admitted to hospital are also likely to suffer significant health care problems.

One of the difficulties in developing strategies to treat traumatic brain injury is the highly heterogeneous nature of both the initial injury and its subsequent pathological development. Severe life-threatening head trauma will inevitably involve mechanisms of developing injury which differ from those that occur following a mild contusion. Nonetheless, a number of common neuronal and biochemical mechanisms are thought to be involved. It seems to be generally agreed that, while the primary injury will cause immediate mechanical damage to both the vasculature and to neuronal tissue, there follows a complex series of interacting injury cascades driven by, among other things, ischemia, cerebral edema and axotomy. The fact that these processes lead to a developing "secondary" injury has given some hope that interventions can be devised which arrest the development of injury or minimize its impact.

Xenon is a noble (and thus generally chemically inert) gas whose anesthetic properties have been known for over 50 years. Since the discovery that xenon is an effective antagonist of NMDA receptors, there has been growing interest in its potential use as a neuroprotectant. Xenon has been shown to reduce neuronal injury in a variety of in vitro and *in vivo* models, and has a number of attractive features, including the fact that it can be rapidly introduced into the brain and cannot be metabolized. It has been shown to be effective in models that involve hypoxia and/or ischemia.

WO-A-01/08692 discloses the use of xenon as a neuroprotectant, inhibitor of synaptic plasticity, and NMDA receptor antagonist. It is indicated that NMDA receptor activation is a result of hypoxia and ischaemia following head trauma, stroke and cardiac arrest, and that NMDA receptor antagonists are neuroprotective under many clinically relevant circumstances, including ischemia, brain trauma, neuropathic states, and certain types of convulsions.

WO-A-03/092707 discloses the use of xenon for the control of neurological deficits associated with cardiopulmonary bypass.

WO-A-05/003253 discloses the use of xenon in the preparation of a medicament for treating, preventing and/or alleviating one or more anesthetic induced neurological deficits.

EP-A-1552840 discloses xenon/carbon monoxide mixtures for use in protecting cells. Suggested clinical indications include cell protection during myocardial infarction, stroke, and heart surgery. Other gases may be added to the mixture, including oxygen, nitrogen, air, and noble gases, such as helium. Helium, if present, is used as a carrier for the more voluminous xenon.

WO-A-06/018655 discloses the use of xenon for treating and/or preventing and/or alleviating one or more anesthetic-induced neurological deficits in a neonatal subject. In one embodiment, the gaseous medicament may further comprise oxygen, nitrogen, air, helium, NO, CO, CO₂, N₂O, argon, krypton, or other gaseous compounds or inhaleable medicaments. Binary mixtures of xenon and oxygen or ternary mixtures of xenon and oxygen and nitrogen are preferred.

WO-A-04/082729 describes the use of a gaseous heat carrier medium which can be cooled and inhaled to reduce body temperature in order to provide neuroprotection in a number of clinical scenarios, including traumatic brain damage and severe head injury. The heat carrier medium comprises a gas having a density of more than 6g/L at 0°C and atmospheric pressure, which dense gas is preferably a perfluorocarbon. The dense gas is typically present at a concentration of between 20 and 80%. The heat carrier medium may additionally comprise other gases selected from oxygen, nitrogen, carbon dioxide, carbon monoxide, nitrous oxide, nitric oxide, argon, helium, gaseous hydrocarbons, and xenon. Helium, where present, is used to provide greater inspired gas temperature equalisation towards body temperature.

US-A-2005/0152988 discloses the use of argon to prevent or treat neurointoxication in humans, including treatment of acute cerebral accidents. The mixtures used may consist of argon and oxygen only, or may include also other gases such as xenon, krypton, nitrogen, nitrous oxide, nitrogen or air.

The present invention relates to the use of helium for providing neuroprotection.

Whilst the neuroprotective properties of xenon are well known, it has now surprisingly been found that administration of helium can provide a significant neuroprotective effect. So far as the inventors are aware, the ability of helium to provide neuroprotection has not previously been suggested or predicted.

Thus, according to a first aspect the present invention provides a gaseous mixture consisting of helium, oxygen, optionally nitrogen, and optionally carbon dioxide, for use as an inhaled medicament for providing neuroprotection. As the mixture consists of the aforementioned gases, the presence of other gases is, in general, prohibited. However, trace amounts of other gases may be present.
Thus, for example, a suitable gaseous mixture may be obtained from air by admixing helium with air or oxygen enhanced air (helium needs to be added, as the levels of helium in atmospheric air are negligible).

As used herein, the term "neuroprotection" means protecting a neural entity, such as a neuron, at a site of injury, for example an ischemic or traumatic injury.

The term "inhaled medicament" means a medicament for administration by inhalation or simulated inhalation. The term "simulated inhalation" refers to those situations where a patient is or may be unable to breath unassisted, and is therefore placed on to a heart-lung machine (also known as a pump oxygenator or cardiopulmonary bypass machine) or similar device. In such circumstances, the gaseous medicament is administered to the oxygenator of the heart-lung machine, which simulates the function of the patient's lungs in allowing oxygen (and the noble gas) to diffuse into (and carbon dioxide to diffuse out of) blood drawn from the patient. The oxygen enriched blood is then pumped back to the patient.

In a preferred embodiment, the neuroprotection is against neuronal damage resulting from an impact trauma.

In a preferred embodiment, the gaseous mixture has a nitrogen content of equal to or less than about 60%. More preferably the nitrogen cotent is equal to or less than 40%, 20%, or 10% by volume. Most preferably, the gaseous mixture is essentially free of nitrogen. The helium content of the gaseous mixture is preferably equal to or more than about 20%. More preferably the helium content is equal to or more than 40%, 60%, or 70% by volume. Most preferably, the gaseous mixture has a helium content of about 80% by volume, such as in the range of 75 to 85%.
The oxygen content of the gaseous mixture is preferably about 20%, such as in the range of 15 to 25%.

The inventors found that nitrogen appears to exacerbate neuronal injury, and it is therefore preferred that the presence of this gas is minimised, for example by replacing nitrogen, in air which would normally be inhaled by a patient, with helium.

In a preferred embodiment the medicament is for administration in a normobaric environment. The terms "hyperbaric" and "normobaric" have their ordinary meaning in the art, i.e. normobaric means a pressure equal to about 1 atm (normal air pressure at sea level; approximately 0.1 MPa) and hyperbaric means a pressure above normobaric pressure.

In such an embodiment, therefore, the administered medicament has a nitrogen partial pressure of equal to or less than about 0.6 atm (0.06 MPa)(corresponding to a gas mixture having a nitrogen content of equal to or less than about 60%, when administered under normobaric conditions). More preferably still, the administered medicament has a nitrogen partial pressure of equal to or less than about 0.4 atm (0.04 MPa). Most preferably the gas mixture is essentially free of nitrogen.

Likewise, the administered medicament preferably has a helium partial pressure of equal to or more than about 0.2 atm (0.02 MPa)(corresponding to a gas mixture having a helium content of equal to or more than about 20%, when administered under normobaric conditions), more preferably equal to or more than about 0.4 atm (0.04 MPa), and most preferably about 0.8 atm (0.08 MPa).

Similarly, the oxygen partial pressure in the administered medicament is preferably about 0.2 atm (about 0.02 MPa, i.e. the same as in normobaric air, corresponding to a gas mixture having a oxygen content of about 20% when administered under normobaric conditions).

According to a second aspect the present invention provides a gaseous mixture comprising about 80% (such as in the range of 75 to 85%) by volume helium and about 20% (such as in the range of 15 to 25%) by volume oxygen, wherein helium is the sole noble gas, for use as an inhaled medicament for providing neuroprotection. As helium is the sole noble gas, the presence of other noble gases is in general prohibited, although it may again be the case that trace amounts of other noble gases are present, as for example may arise where the gaseous mixture is obtained from air. The noble gases consist of those elements found under Group 18 of the periodic table, i.e. the currently known noble gases are helium, neon, argon, krypton, xenon and radon.

As with the first aspect, in preferred embodiments the neuroprotection is against neuronal damage resulting from an impact trauma, and/or the medicament is for administration in a normobaric environment.

According to a third aspect, the present invention provides the use of helium for the manufacture of a gaseous inhaled medicament for providing neuroprotection, said gasesous medicament consisting of helium, oxygen, optionally nitrogen, and optionally carbon dioxide.

According to a fourth aspect, the present invention provides a method of providing neuroprotection comprising administering as an inhaled medicament to a patient in need of neuroprotection a gaseous mixture consisting of helium, oxygen, optionally nitrogen, and optionally carbon dioxide.

The features and preferred embodiments of the third and fourth aspects of the present invention are as described above in relation to the first aspect of the invention.

According to a fifth aspect, the present invention provides the use of helium for the manufacture of a gaseous inhaled medicament for providing neuroprotection, said gasesous medicament comprising about 80% by volume helium and about 20% by volume oxygen, wherein helium is the sole noble gas in the gaseous medicament.

According to a sixth aspect, the present invention provides a method of providing neuroprotection comprising administering by inhalation to a patient in need of neuroprotection a gaseous mixture comprising about 80% by volume helium and about 20% by volume oxygen, wherein helium is the sole noble gas.

The features and preferred embodiments of the fifth and sixth aspects of the present invention are as described above in relation to the second aspect of the invention.

The invention is further described below by way of nonlimiting example, with reference to the accompanying figures in which:
Figure 1 depicts, both in overview (A) and in a close-up view of part thereof (B), an experimental apparatus used to induce reproducible focal injury in organotypic brain slices;
Figure 2 is a graph showing the distribution of fluorescent intensity for two propidium iodide (PI) stained hippocampal slices, one with and one without focal trauma, 72 hours after trauma - also shown (inserts) are fluorescent images of a hippocampal slice without trauma (upper left) and with trauma (lower left), and a graph (upper right) of propidium iodide staining of cells permeabilized with 70% ethanol;
Figures 3A and B are bar charts showing (A) the development of injury including the site of focal injury ("Total injury") and (B) the development of injury excluding the site of focal injury ("Secondary injury");
Figure 4 is a graph showing the effects of added pressures of helium and nitrogen on the development of total injury;
Figure 5 is a graph showing the effects of added pressures of xenon on the development of total injury; and
Figure 6 is a bar chart showing the effects on the development of total injury of substituting normobaric air with a normobaric gas containing helium or xenon in an amount equivalent to the nitrogen content of normobaric air.

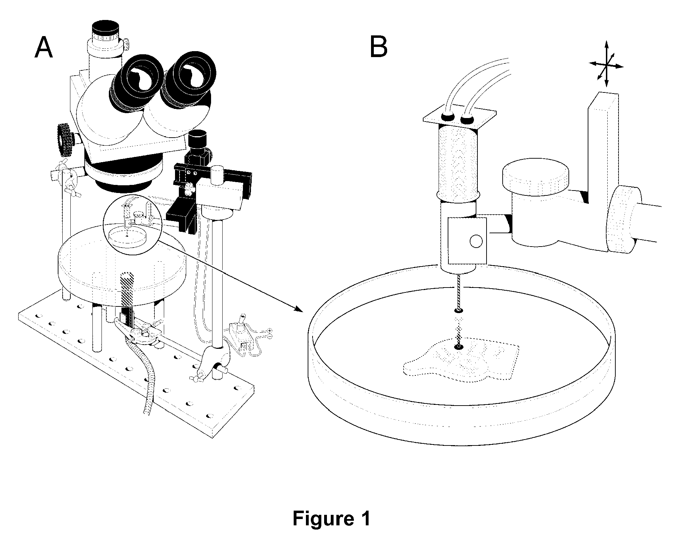

The properties of xenon and other gases were investigated in an *in vitro* model of traumatic brain injury (as described below in greater detail, under the heading "Example"). The model chosen involved creating a focal mechanical trauma centered on the CA1 region of cultured hippocampal brain slices, and assessing neurological injury using propidium iodide staining. The apparatus used to induce focal injury in the organotypic brain slices is shown in Figure 1. A small solenoid retains a stylus 5 mm above a cultured hippocampal slice which is positioned using a micromanipulator. A fiber-optic light source illuminates the slice from beneath. As shown in (B) the stylus is constrained in a glass capillary and positioned just above the CA1 region of the hippocampus.

Hippocampal brain slices after two weeks in culture, so called organotypic slices, maintain heterogeneous populations of cells whose synaptic contacts reflect, at least to some extent, the *in vivo* state. They represent a useful compromise between models that use dissociated cell cultures and those that use intact animals. The nature of the focal trauma, and the subsequent slowly developing secondary injury, bears a sufficiently close relationship to the *in vivo* situation to provide a useful model in which to test possible treatments.

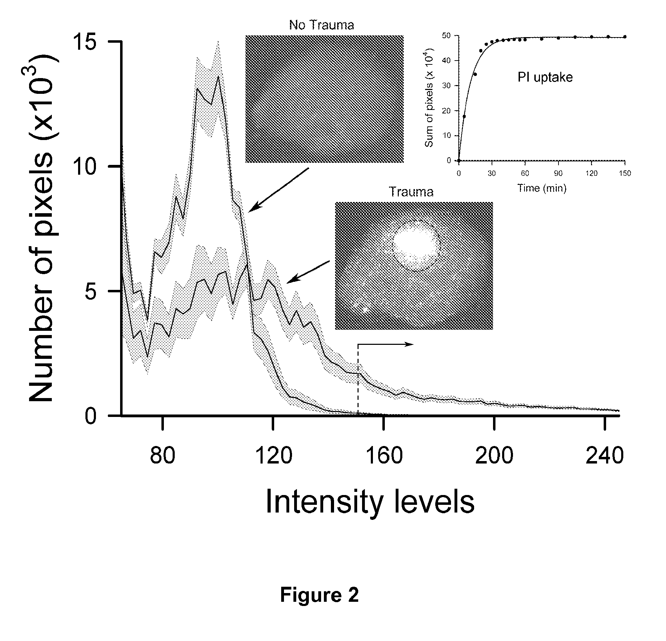

Figure 2 shows the distribution of fluorescent intensity for two propidium iodide (PI) stained hippocampal slices. One slice was subject to a focal trauma and one what not, the images being taken 72 hours after trauma. PI is a membrane-impermeable dye that can only enter cells with damaged cell membranes, that becomes highly fluorescent on binding to DNA, and that can therefore be used to quantify cell injury in terms of fluorescence. As is illustrated in Figure 2, trauma caused a marked rightwards shift in the intensity distribution.

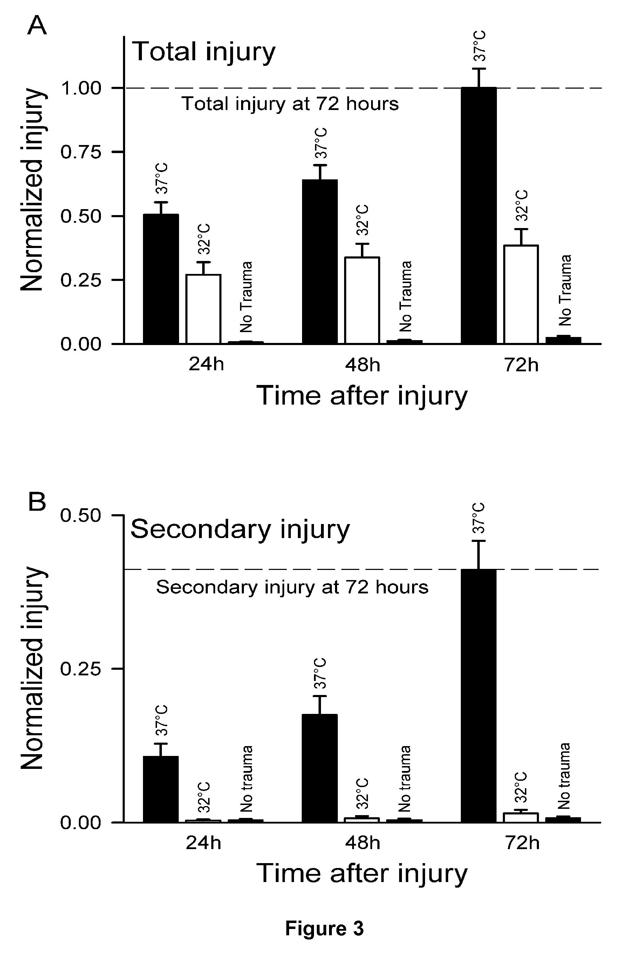

Figures 3A and B chart the development of injury in the hippocampal slices over time. Figure 3A shows the development of injury including the site of focal injury ("Total injury") and Figure 3B shows the development of injury excluding the site of focal injury ("Secondary injury"). The solid bars represent the data obtained for slices maintained at 37°C while the open bars represent the data obtained for slices maintained at 32°C. The bars labeled "no trauma" represent data from brain slices which have not suffered traumatic injury. The dashed line in Figure 3A represents the total injury under control conditions after 72h (at 37°C), which has been normalized to unity (as described infra in greater detail). The error bars are SEMs and the data are from an average of 12 slices.

Within hours of injury being induced bright punctate propidium iodide staining was evident, a pattern of staining that has been shown to correlate well with neuronal damage assessed by morphological changes. The injury slowly increased with time and approximately doubled between 24h and 72h (Figure 3A). When the region of the slice centered at the focal point of injury was excluded from the analysis, the development of injury was even more dramatic. This secondary injury distant from the site of mechanical trauma, which starts from baseline levels, progressively increased (Figure 3B).

The difference between this secondary injury and the total injury which included the region close to the focal point of trauma was particularly evident when the effects of subjecting the brain slices to temperatures equivalent to moderate hypothermia were investigated. When the brain slices were incubated at 32°C following trauma, the secondary injury was almost completely eliminated (Figure 3B). In contrast, this level of hypothermia reduced the total injury by a more limited extent (Figure 3A), presumably because the initial mechanical injury that was caused by the focal trauma was irreversible.

The neuroprotective effects of hypothermia are well known and have been shown in a variety of laboratory models of injury. Indeed, a similar observation has been made before using an equivalent model to the one employed herein, although the finding that hypothermia is much more effective against secondary rather than primary injury goes a step further than the findings in the earlier study.

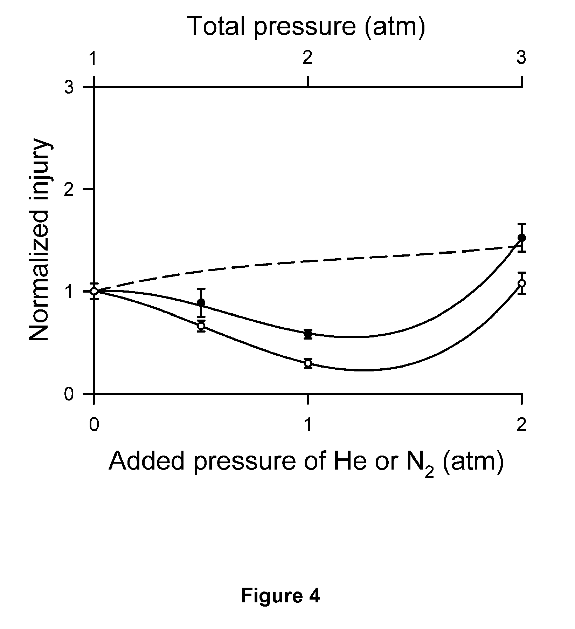

Having established a test protocol that produced a consistent and reproducible injury, the effects of varying other factors were investigated. Most surprisingly, it was found that subjecting the brain slices to added pressures of helium (adding helium to normobaric air to provide a hyperbaric environment) provided potent neuroprotection (Figure 4). Figure 4 shows the effects of added pressures (pressure added to 1 atm (0.01 MPa) of air) of helium and nitrogen on the development of total injury. The effects of helium are shown as open circular symbols and those of nitrogen are shown as filled circular symbols. The solid lines are drawn by eye. Error bars are SEMs for an average of 14 slices.

The effects of added helium are considered to be the effects of added pressure *per* se for a number of reasons. It is widely accepted that even at high pressures, helium is unlikely to exert any direct pharmacologic effects, due to its very low solubility in biological tissues. Indeed, when the effects of helium have been directly compared to those of hydrostatic pressure, it has almost invariably been concluded that any observed effects are due to pressure *per se.* The excitable effects of high pressures (the so-called high pressure nervous syndrome) are well known among divers, but these effects generally occur at pressures slightly above those used in the present investigations. Nonetheless, significant effects on neuronal excitability have been described using *in vitro* systems over the pressure ranges used herein. While the effects of pressure are complex, one consistent finding is that excitatory synapses are depressed, most likely because of suppression of neurotransmitter release. Reductions in the levels of glutamate could be neuroprotective because they would tend to mitigate against excitotoxicity and, without being bound by any theory, this may be a basis for the neuroprotection observed at up to 2 atmospheres (0.2 MPa) of added pressure.

Given that helium at these low pressures is unlikely to be exerting any effects on its own, the significantly worse injury outcome that occurred when helium was replaced by nitrogen (Figure 4) is probably due to some deleterious effect of nitrogen *per se.* The dashed line in Figure 4 is constructed by subtracting the effects of helium (considered to be the effects of pressure *per se)* from the effects of nitrogen to give the theoretical effect of increasing nitrogen levels *per se* (i.e. excluding the concurrent effects of increased pressure).

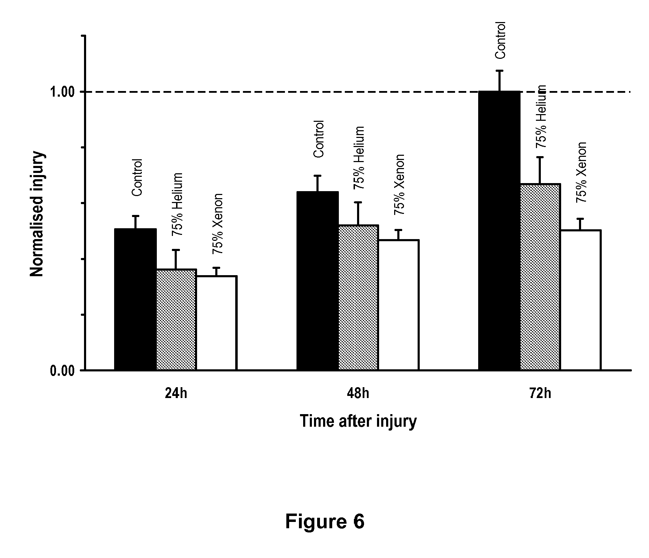

As nitrogen has a considerably higher fat solubility than helium (implying a higher partitioning into brain tissue), and as the effects of nitrogen narcosis are evident at only a few atmospheres, it is perhaps not surprising that even low pressures of nitrogen exert some pharmacologic effects, although it could not have been predicted whether these would have been beneficial or harmful. If nitrogen is indeed deleterious, then its replacement by helium should be neuroprotective even at normobaric pressures, and by an extent inverse to the deleterious effects that can be predicted from the dashed line in Figure 4 of a normobaric partial pressure of nitrogen. When this experiment was performed the observed a level of injury (after 72 hours) in a brain slice exposed to normobaric air with helium substituted for nitrogen (0.67 ± 0.10, see Figure 6) was indeed very close to that predicted theoretically (0.75).

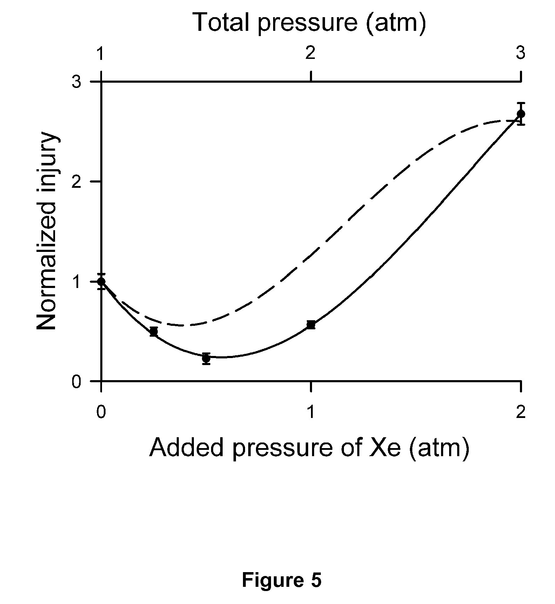

The effects of added pressures of xenon on the development of total injury were also investigated, the results being shown in Figure 5. The dashed line in Figure 5 is constructed by subtracting the effects, as depicted in Figure 4, of helium (assumed to be those of pressure *per se*) from the effects of xenon to give the theoretical effect of increased xenon levels *per se* (i.e. excluding the concurrent effects of increased pressure). Error bars are SEMs for an average of 13 slices. As the data in Figure 5 shows, xenon exhibited marked neuroprotection at low pressures, but then showed toxicity at the highest pressure used. Both of these features, neuroprotection and toxicity, are due to xenon itself as shown by dashed line in Figure 5. Xenon toxicity has not been previously reported, although to the inventors' knowledge such high levels of xenon have not previously been tested.

Finally, the effects of substituting helium and xenon for the nitrogen content of normobaric air was investigated in further detail (i.e. replacing normobaric air with a normobaric mixture of 25% O₂, 5% CO₂, and either 75% Xe or 75% He), the results of which are shown in Figure 6. As might be expected from the results obtained in the hyperbaric tests, both the substitution of nitrogen with helium and the substitution of nitrogen with xenon produced marked reductions in injury, which were evident after 24 hours, and became more pronounced as further time passed. Xenon was found to exhibit a slightly greater neuroprotective effect that helium, as would be expected based on the beneficial pharmacological effects of 0.75 atm of xenon predicted from the dashed line in Figure 5. However, the use of helium also provided significant neuroprotective effects, as compared to normobaric air.

Further details of the investigations described above are provided below.

### EXAMPLE

### Materials and Methods

Unless otherwise stated, all chemicals were obtained from Sigma Chemical Company Ltd. (Poole, Dorset, UK.)

### Hippocampal organotypic slices

Organotypic hippocampal slice cultures were prepared as reported by Stoppini L, et al in "A simple method for organotypic cultures of nervous tissue" J Neurosci Methods 1991;37:173-82, subject to some modifications. Briefly, brains were removed from seven-day-old C57/BL6 mice pups (Harlan UK Ltd., Bicester, Oxfordshire, UK) and placed in ice-cold "preparation" medium. The preparation medium contained Gey's balanced salt solution and 5 mg ml⁻¹ D-glucose (BDH Chemicals Ltd., Poole, Dorset, UK). The hippocampi were removed from the brains and 400µm thick transverse slices were prepared using a McIllwain tissue chopper. Slices were transferred into ice-cold preparation medium, gently separated and then placed onto tissue culture inserts (Millicell-CM, Millipore Corporation, Billerica, MA) which were inserted into a six-well tissue culture plate. The wells contained "growth" medium which consisted of 50% Minimal Essential Media Eagle, 25% Hank's balanced salt solution, 25% inactivated horse serum, 2 mM L-glutamine, 5 mg ml⁻¹ D-glucose (BDH) and 1% antibiotic-antimycotic suspension. Slices were incubated at 37°C in a 95% air/5% CO₂ humidified atmosphere. The growth medium was changed every three days. Experiments were carried out after 14 days in culture.

### Traumatic injury and hyperbaric gas chamber

After the hippocampal slices had been in culture for 14 days, the growth medium was changed to "experimental" medium. The experimental medium was serum-free and consisted of 75% Minimal Essential Media Eagle, 25% Hank's balanced salt solution, 2 mM L-glutamine, 5 mg ml⁻¹ D-glucose, 1% antibiotic-antimycotic suspension and 4.5 µM propidium iodide.

The trauma to the slices was produced with a specially designed apparatus (Figure 1) which was based on published descriptions (Adamchik Y, et al "Methods to induce primary and secondary traumatic damage in organotypic hippocampal slice cultures" Brain Res Brain Res Protoc 2000;5:153-8 and Adembri C, et al "Erythropoietin attenuates post-traumatic injury in organotypic hippocampal slices" J Neurotrauma 2004;21:1103-1220,21). Under a stereomicroscope a stylus was positioned 5 mm above the CA1 region of the hippocampus using a three-axis micromanipulator. The stylus dropped onto the slice with an impact of 3.5 µJ when power to a small electromagnet was switched off. This energy was selected so that the stylus dropped onto the tissue and did not rebound; this produced a consistent and reproducible focal traumatic injury. The distal part of the stylus was smooth and rounded in order to prevent perforation of the slice and the impact produced a focal injury with a diameter of 750 ± 17 µm (mean ± SD).

After traumatizing the CA1 region, the culture trays were transferred to a small pressure chamber which contained a high-speed fan for rapid gas mixing. The pressure chamber was capable of maintaining a constant pressure of up to six atmospheres for several days. The pressure chamber was housed in an incubator which was set at 37°C for normothermic experiments or 32°C for experiments at moderate hypothermia. Subsequently, the pressure chamber (gas volume 0.925 litre) was flushed with humidified control gas (95% air and 5% CO₂) for 5 min at 5 litres min⁻¹ which ensures better than 99.99% gas replacement. After flushing, the pressure chamber was sealed, and slices under these conditions were considered to be the "injury controls" (75% nitrogen/20% oxygen/5% CO₂).

For the experiments under hyperbaric conditions, the chamber was pressurized with experimental gas (xenon, helium or nitrogen between 0.25 atm (0.025 MPa) and 2 atm (0.2 MPa), added *in addition* to the 1 atmosphere (0.1 MPa) of 95% air/5% CO₂ and then sealed. To test the effects of helium and xenon under normobaric conditions, the pressure chamber was flushed with humidified gas mixtures containing either 75% helium/20% oxygen/5% CO₂ or 75% xenon/20% oxygen/5% CO₂ for five minutes and then sealed.

After 24h in the chamber, the slices were imaged using a fluorescent microscope (as described in greater detail below). After completing the imaging, the slices were transferred back to the pressure chamber and the appropriate gas mixture and pressure re-established. This procedure was repeated at 48h and 72h post trauma. It should be noted that, for all gas mixtures and for all pressures, the partial pressures of oxygen and carbon dioxide were fixed at 0.2 atm (0.02 MPa) and 0.05 atm (0.005 MPa), respectively.

### Quantifying cell injury

Propidium iodide (PI) is a membrane-impermeable dye that only enters cells with damaged cell membranes. Inside the cells it binds principally to DNA and becomes highly fluorescent, with a peak emission spectrum in the red region of the visible spectrum. An epi-illumination microscope (Nikon Eclipse 80, Kingston upon Thames, Surrey, UK), and a low-power (2x) objective were used to visualize the PI fluorescence. A digital video camera and software (Micropublisher 3.3 RTV camera and QCapture Pro software, Burnaby, British Columbia, Canada) were used to capture the images. The images were analyzed using the ImageJ software (http://rsb.info.nih.gov). Red, green and blue channels were recorded, but only the red channel was used and the distribution of intensities was plotted as a histogram with 256 intensity levels.

Slices under standard control conditions (incubated in the chamber for 72h at 37°C with 95% air and 5% CO₂) showed a well-defined peak in the intensity distribution (Figure 2) which fell rapidly to zero. In contrast, following trauma, the peak in the intensity distribution was lower, broader and shifted to higher intensity levels (Figure 2).

As a measure of trauma, the number of pixels above an intensity threshold of 150 were integrated (indicated by the arrow and dashed line in Figure 2), which under the experimental conditions used provided a robust quantitative measurement of PI fluorescence, and hence of cell injury. Injury could then be expressed relative to the total injury observed after 72h under control conditions (75% nitrogen, 20% oxygen, and 5% CO₂; 1 atm (0.1 MPa); and 37°C), which was normalized to unity. Thus, for example, if under a particular set of test conditions the integrated number of pixels above an intensity threshold of 150 (calculated as above) is half that present under control conditions, the injury produced by those test conditions is characterized as 0.5 the normalized injury.

Two different measures of injury were used: "total" injury, which was defined as the increase in fluorescence over the entire slice; and "secondary" injury, which was the increase in fluorescence over the slice but excluding the region covering the focal injury. The region covering the focal injury was excluded by masking the area of focal injury in the image prior to integration. The mask was a circle with a diameter of 1000 mm (outline depicted by the dotted circle in the lower image in Figure 2), which was sufficiently large to cover the region of focal injury.

Because the light output from the mercury lamp changed over time, the exposure time was adjusted to take this into account. This was done by recording fluorescence from a glass slide standard (Fluor-Ref, Omega Optical, Brattleboro, VT) and adjusting the exposure time accordingly.

### Results

Within three hours of producing the focal injury, increased fluorescence at the site of injury was evident, indicating almost immediate cell injury in this region. The fluorescence intensity continued to slowly increase, however, both within the region of focal injury as well as over regions of the brain slice distant from the site of injury ("secondary" injury). The slowly developing increase in PI fluorescence was due to increasing cell injury rather than increasing PI binding to cells that have already died. This can be deduced from the data in the inset to Figure 2 which shows that when cells are permeabilized using ethanol, PI binding is very rapid (with a half-time of about 7 minutes), and that equilibration is complete after approximately 30 minutes. In contrast, cell injury following trauma progresses slowly and continues to increase for at least 72h post injury, the longest time point investigated.

The filled bars in Figure 3A show the increase in total injury at 37°C with time, normalized to the injury observed at 72h, while the filled bars in Figure 3B show injury for the same slices but where the focal injury has been excluded from the analysis. The damage in the absence of traumatic injury (labeled "no trauma") was negligible at all time points. A comparison of the data in Figures 3A and B show that the secondary injury constitutes an increasing proportion of the total injury as time progresses.

Moderate hypothermia (32°C) greatly reduced the development of injury after 24h. The open bars in Figure 3A show that the development of total injury with time is very modest at 32°C and therefore, proportionately, the protection due to hypothermia became greater with increasing time. For example, at 24h, hypothermia reduced total injury by about 46% while at 72h, hypothermia reduced injury by 62%.
Figure 3B shows the effects of hypothermia on secondary injury. When the focal site of injury is excluded, it can be seen that the effects of hypothermia in reducing injury are even more pronounced. At 72h, for example, injury is reduced by over 96%.

Having established a protocol that produced a consistent and reproducible injury, the effects of the noble gas helium on the development of injury were investigated. The results are presented in Figure 4, where the open circular symbols show that for pressures up to about 1 atm (0.1 MPa) of added helium, injury decreased, but as higher pressures of helium were added, the injury worsened and came back close to control levels at an added pressure of about 2 atm (0.2 MPa; about 3 atm (0.3 MPa) total pressure). The experiments were then repeated, except with nitrogen substituted for helium. Qualitatively the effects of nitrogen were very similar (filled circular symbols in Figure 4) to those observed with helium, although at all pressures the outcome was significantly worse.

Because helium is most unlikely to be exerting a pharmacologic effect at these low pressures it is reasonable to conclude that the effects observed with helium are due to pressure *per se.* On this basis, the theoretical effect of increased nitrogen levels (independent of the effects of pressure) was calculated (dashed line in Figure 4) by subtracting the effects of helium (constituting the effects of added pressure *per se)* from the effects of added pressures of nitrogen. Nitrogen can thus be seen to be detrimental in a fashion that is roughly linear with increasing amounts. It follows that if helium replaced nitrogen in air under normobaric conditions then this would be predicted to be neuroprotective also, if only because it was replacing the deleterious effects of the nitrogen normally present in the air. This prediction was tested by using a mixture of 75% helium/20% oxygen/5% CO₂, from which it was found that the observed degree of normalized injury after 72 hrs (0.67 ± 0.10; Figure 6) was indeed close to that predicted simply by the absence of nitrogen (0.75).

Next, the effects of xenon, which has as noted above been shown to have neuroprotective properties in a variety of in vitro and *in vivo* models of neuronal injury, were investigated. The results are presented in Figure 5, and show that low added pressures of xenon provided marked neuroprotection but that this was reversed at the highest pressure tested where significant neurotoxicity was observed.

Following the logic outlined above, the theoretical effect of increased xenon levels (independent of the effects of pressure) can also be calculated by subtracting the effects of helium (concluded to be the effect of pressure *per se).* The result of this calculation is shown as the dashed line in Figure 5. The validity of this analysis can also be tested by measuring the neuroprotection afforded by 75% xenon under normobaric conditions. The protection that would be predicted would be that expected of this level of xenon (calculated from the dashed line in Figure 5), plus the benefit of removing the deleterious effect of nitrogen at the same level (calculated from the dashed line in Figure 4) as in this case xenon would be replacing nitrogen. The predicted level of injury after 72 hrs is calculated to be 0.59 and the observed degree of injury was found to be 0.50 ± 0.04 (Figure 6), a reasonable agreement between predicted and experimental values.

Finally, the effects of normobaric mixtures of 25% oxygen, 5% carbon dioxide, and either 75% xenon or 75% helium, were tested to further investigate the effects of substituting the nitrogen content of air, under normobaric conditions, with helium or xenon. The results are shown in Figure 6. As might be expected from the results obtained in the hyperbaric tests, both the substitution of nitrogen with helium and the substitution of nitrogen with xenon produced marked reductions in injury, which were evident after 24 hours and became more pronounced as further time passed. Xenon was found to exhibit a slightly greater neuroprotective effect that helium, but the use of helium also provided significant neuroprotective effects as compared to normobaric air.

Whilst the invention has been described with reference to various specific embodiments, it will be appreciated that variations and modification made be made without departing from the spirit and scope of the invention.

## Claims

1. A gaseous mixture consisting of helium, oxygen, optionally nitrogen, and optionally carbon dioxide, for use as an inhaled medicament for providing neuroprotection.

2. A mixture as claimed in claim 1, wherein the gaseous mixture has a nitrogen content of equal to or less than about 60% by volume and a helium content of equal to or more than about 20% by volume.

3. A mixture as claimed in any preceding claim, wherein the gaseous mixture is essentially free of nitrogen.

4. A mixture as claimed in any preceding claim, wherein the gaseous mixture has a helium content of about 80% by volume.

5. A mixture as claimed in any preceding claim, wherein the gaseous mixture has an oxygen content of about 20% by volume.

6. A gaseous mixture comprising about 80% by volume helium and about 20% by volume oxygen, wherein helium is the sole noble gas, for use as an inhaled medicament for providing neuroprotection.

7. A mixture as claimed in any preceding claim, wherein the neuroprotection is against neuronal damage resulting from an impact trauma.

8. A mixture as claimed in any preceding claim, wherein the medicament is for administration in a normobaric environment.

9. Use of helium for the manufacture of a gaseous inhaled medicament for providing neuroprotection, said gasesous medicament consisting of helium, oxygen, optionally nitrogen, and optionally carbon dioxide.

10. A use as claimed in claim 9, wherein the gaseous medicament has a nitrogen content of equal to or less than about 60% by volume and a helium content of equal to or more than about 20% by volume.

11. A use as claimed in claim 9 or 10, wherein the gaseous medicament is essentially free of nitrogen.

12. A use as claimed in any one of claims 9 to 11, wherein the gaseous medicament has a helium content of about 80% by volume.

13. A use as claimed in any one of claims 9 to 12, wherein the gaseous medicament has an oxygen content of about 20% by volume.

14. Use of helium for the manufacture of a gaseous inhaled medicament for providing neuroprotection, said gasesous medicament comprising about 80% by volume helium and about 20% by volume oxygen, wherein helium is the sole noble gas in the gaseous medicament.

15. A use as claimed in any one of claims 9 to 14, wherein the neuroprotection is against neuronal damage resulting from an impact trauma.

16. A use as claimed in any one of claims 9 to 15, wherein the medicament is for administration in a normobaric environment.

17. A method of providing neuroprotection comprising administering as an inhaled medicament to a patient in need of neuroprotection a gaseous mixture consisting of helium, oxygen, optionally nitrogen, and optionally carbon dioxide.

18. A method as claimed in claim 17, wherein the gaseous mixture has a nitrogen content of equal to or less than about 60% by volume and a helium content of equal to or more than about 20% by volume.

19. A method as claimed in claim 17 or 18, wherein the mixture is essentially free of nitrogen.

20. A method as claimed in any one of claims 17 to 19, wherein the mixture has a helium content of about 80% by volume.

21. A method as claimed in any one of claims 17 to 20, wherein the gaseous mixture has an oxygen content of about 20% by volume.

22. A method of providing neuroprotection comprising administering by inhalation to a patient in need of neuroprotection a gaseous mixture comprising about 80% by volume helium and about 20% by volume oxygen, wherein helium is the sole noble gas.

23. A method as claimed in any one of claims 17 to 22, wherein the neuroprotection is against neuronal damage resulting from an impact trauma.

24. A method as claimed in any one of claims 17 to 23, wherein the method is carried out in a normobaric environment.
